# FASCICULE DE BREVET EUROPEEN

(11) **EP 2 874 670 B1**
(45) Date de publication et mention de la délivrance du brevet: **12.10.2016**
(21) Numéro de dépôt: 13744740.5
(22) Date de dépôt: 15.07.2013
(51) Int. Cl.: A61L 15/24, A61L 15/42, A61L 15/50

(54) **PANSEMENT INTERFACE AUTOPORTE**
SELBSTTRAGENDER SCHNITTSTELLENVERBAND
SELF-SUPPORTING INTERFACE DRESSING

(30) Priorité: 17.07.2012 FR 1256909
(43) Date de publication de la demande: 27.05.2015
(73) Titulaire: Urgo Recherche Innovation et Développement, 21300 Chenove (FR)
(72) Inventeur: AUGUSTE, Stéphane, 21490 Ruffey Les Echirey (FR); DANEROL, Anne-Sophie, 21000 Dijon (FR); LABORDE, Aurélie, 21160 Marsannay La Côte (FR); DESMAISON, Nadège, 21110 Tart Le Haut (FR)
(74) Mandataire: Hubert, Philippe
(86) Numéro de dépôt international: PCT/FR2013/051690
(87) Numéro de publication internationale: WO 2014/013175

(56) Documents cités:
- EP-A1- 0 521 761
- WO-A1-01/70285
- WO-A2-00/16725
- FR-A1- 2 936 158

## Description

La présente invention concerne un pansement interface autoporté destiné au traitement des plaies.

Dans le cadre du traitement des plaies, on utilise depuis de nombreuses années des pansements destinés à être appliqués directement sur la plaie en assurant une interface entre ladite plaie et une compresse absorbante placée sur ledit pansement afin d'absorber les exsudats de la plaie. De tels pansements sont habituellement désignés par l'expression « pansements interface ».

Parmi les pansements interface actuellement sur le marché, on peut citer par exemple le produit commercialisé par la société Laboratoires URGO sous la dénomination Urgotul^{®}.

Ce produit, qui est notamment décrit à l'exemple 1 du document WO 00/16 725 est généralement constitué d'une armature faite d'un tissu à mailles ouvertes dont les fils sont enrobés par un gel cohésif et non-adhérent, de façon à laisser les mailles essentiellement non-obturées.

Ce gel est formé d'une matrice élastomérique hydrophobe fortement plastifiée et contenant en dispersion une faible quantité de particules hydrophiles d'un hydrocolloïde.

Le pansement Urgotul^{®} est particulièrement avantageux dans la mesure où il n'adhère pas aux tissus nouvellement régénérés et maintient des conditions d'humidité optimales favorables à la cicatrisation, tout en évitant le risque de macération de la plaie.

Cependant, en raison de la rigidité de son armature, le produit Urgotul^{®} manque de conformabilité et ne peut être utilisé de façon aisée pour le traitement de plaies difficilement recouvrables en raison de leur localisation, comme par exemple les doigts de la main ou les articulations comme le coude.

Dans ce contexte, il serait souhaitable de disposer d'un pansement interface qui présenterait les mêmes avantages que le produit Urgotul^{®}, mais qui serait exempt d'armature, c'est-à-dire autoporté. Un tel pansement devra présenter une rigidité suffisante pour être facilement manipulé et appliqué sur une plaie.

Cependant, la réalisation d'un tel produit, qui doit satisfaire des propriétés parfois antagonistes, est particulièrement complexe.

En effet, ce produit doit être :
- le plus fin possible pour être conformable, tout en présentant une cohésion suffisante pour pouvoir être manipulé sans risque de se déchirer aussi bien lors de sa fabrication que lors de son utilisation ;
- suffisamment extensible dans le sens transversal et dans le sens longitudinal, tout en restant cohésif ;
- de préférence transparent ou translucide pour permettre au personnel soignant de surveiller la cicatrisation de la plaie sans retirer le pansement ;
- facile à fabriquer.

En outre, ce produit doit aussi conserver les propriétés de non adhérence à la peau et à la plaie, le toucher gras mais agréable du produit Urgotul^{®} et garantir la favorisation du processus de cicatrisation et en particulier la prolifération des fibroblastes.

Les compositions des matrices élastomériques utilisées dans la fabrication du pansement Urgotul^{®} et de ses déclinaisons commerciales actuelles ne permettent pas d'obtenir un pansement autoporté, c'est-à-dire sans armature.

Dans le brevet français n° 2 936 158, il est proposé un pansement interface sans armature se présentant sous forme d'une couche mince comprenant des trous traversants permettant le passage des exsudats et constituée d'une matrice hydrophobe spécifique. Cette matrice hydrophobe comprend un élastomère constitué d'un copolymère linéaire tribloc/dibloc de styrène et d'isoprène (SIS/SI), une huile de plastification, un polyéthylène et des particules d'hydrocolloïde dans des proportions relatives précises.

Le pansement interface décrit dans ce brevet français n° 2 936 158 présente de bonnes propriétés de conformabilité, mais ce produit n'est pas entièrement satisfaisant, notamment en raison de la nature de sa matrice constitutive.

En effet, cette matrice élastomérique comporte un copolymère à séquence centrale insaturée (isoprène) sensible à l'oxydation, qui peut donc se dégrader au cours du temps en affectant ainsi les propriétés initiales du produit avec le risque corrélatif d'une moins bonne cicatrisation.

Ce pansement interface n'est pas commercialisé à ce jour.

Ainsi, il existait un besoin de disposer d'un produit plus conformable que le produit Urgotul^{®} qui est commercialisé depuis plus de dix ans, aucun pansement interface autoporté n'a été commercialisé à ce jour.

Dans ce contexte, il a été découvert qu'il était possible de mettre au point un pansement autoporté présentant les mêmes avantages que le produit Urgotul^{®}, en utilisant des matrices élastomériques de compositions beaucoup plus proches de celle du produit Urgotul^{®} que ne le propose le brevet français n° 2 936 158.

EP 0 521 761A1 décrit un pansement autoporté sans trou traversant formé d'une matrice comprenant 10 à 30% en poids d'un copolymère blocs à séquence centrale saturée (SEBS) et 70 à 90% en poids de vaseline.

De même, les matrices élastomériques susceptibles d'être utilisées pour la mise au point du pansement interface selon l'invention ne comportent pas d'élastomère à séquence centrale insaturée, ni de composés additionnels, tels qu'un polyéthylène, comme il est préconisé dans le brevet français précité. De ce fait, ces matrices ne sont pas susceptibles de se dégrader par oxydation au cours du temps et garantissent le maintien des propriétés initiales du pansement.

Plus spécifiquement, les matrices élastomériques utilisées dans le cadre de la présente invention comportent comme seul élastomère, un copolymère tribloc spécifique de type styrène-oléfine saturée-styrène qui présente une viscosité comprise entre 0,2 et 2 Pa.s telle que mesurée dans une solution à 10 % (masse/masse) dans le toluène, ledit élastomère étant présent au sein de la matrice élastomérique en une quantité pondérale prédéterminée et dans des proportions relatives avec les autres composants de la matrice (plastifiant et vaseline) spécifiquement choisies.

Ainsi, selon un premier aspect, la présente invention a pour objet un pansement interface autoporté formé d'une couche mince d'une composition comprenant une matrice hydrophobe et comportant des trous traversants, ladite matrice hydrophobe comprenant :
- pour 100 parties en poids d'un copolymère tribloc styrène-oléfine saturée-styrène présentant une viscosité comprise entre 0,2 et 2 Pa.s, telle que mesurée dans une solution à 10 % (masse/masse) dans le toluène ;
- de 400 à 1 220 parties en poids d'un plastifiant, de préférence une huile plastifiante ; et
- de 0 à 720 parties en poids de vaseline ;
   étant en outre précisé que :
   - la quantité totale de plastifiant et de vaseline est supérieure ou égale à 750 parties en poids ;
   - la quantité de vaseline est comprise entre 400 et 720 parties en poids lorsque la quantité de plastifiant est comprise entre 1 000 et 1 220 parties en poids.

Selon une caractéristique particulière, la matrice hydrophobe précitée comprend en outre des particules d'hydrocolloïdes en une quantité inférieure ou égale à 25 % en poids, rapportée au poids total de ladite matrice hydrophobe.

Selon l'invention, la matrice hydrophobe précitée est généralement constituée d'un élastomère, d'un plastifiant et éventuellement de vaseline.

Cette matrice comporte, à titre de seul élastomère, un copolymère tribloc séquencé comportant deux blocs terminaux thermoplastiques styrène et une séquence centrale élastomérique constituée d'une polyoléfine saturée.

Cette polyoléfine saturée peut être de type poly(éthylène-butylène) ou poly(éthylène-propylène).

Les copolymères triblocs à séquence centrale saturée sont bien connus de l'homme de l'art.

Ces composés présentent des propriétés variées et se différencient, en particulier, par leur viscosité.

Il a été découvert que la réalisation d'un pansement autoporté est subordonnée au choix d'un copolymère de viscosité spécifique.

Ainsi, dans le cadre de la présente invention, l'élastomère sera spécifiquement choisi parmi les copolymères du type présentant un haut poids moléculaire, caractérisés par une viscosité comprise entre 0,2 et 2 Pa.s telle que mesurée dans une solution à 10 % (masse/masse) dans le toluène.

Des copolymères triblocs à séquence centrale saturée répondant à cette propriété sont par exemple commercialisés :
- par la société KRATON POLYMERS sous la dénomination KRATON^{®} G1651 ou KRATON^{®} G1654 pour les copolymères séquencés poly(styrène-éthylène-butylène-styrène)(en abrégé SEBS) ;
- par la société KURARAY sous la dénomination SEPTON^{®} S2006 pour les copolymères séquencés poly(styrène-éthylène-propylène- styrène) (en abrégé SEPS) et SEPTON^{®} S8006 pour les copolymères séquencés SEBS.

Dans le cadre de la présente invention, on préférera les copolymères triblocs SEBS ou SEPS ayant une teneur en styrène comprise entre 25 et 45 % en poids, rapporté au poids total dudit copolymère.

D'une façon préférée, on utilisera les produits commercialisés par la société KRATON POLYMERS sous les dénominations KRATON^{®} G1651 et KRATON^{®} G1654.

Pour obtenir un pansement interface autoporté, non seulement la nature de l'élastomère utilisé doit être spécifiquement choisie, mais en outre, cet élastomère devra être utilisé dans des proportions relatives spécifiques au sein de la matrice hydrophobe, comme on le verra par la suite.

Le plastifiant utilisé pour la réalisation de la matrice hydrophobe est destiné à améliorer les propriétés d'étirement, de souplesse, d'extrudabilité et de mise en oeuvre de l'élastomère précité.

De préférence, ce plastifiant sera constitué d'un liquide ou d'un mélange de liquides compatible avec la séquence centrale polyoléfine saturée de l'élastomère utilisé.

De préférence encore, ce plastifiant est exempt de vaseline et sera choisi parmi les composés qui présentent un point de goutte inférieur ou égal à 35°C.

Parmi les plastifiants susceptibles d'être avantageusement utilisés, on peut citer en particulier les huiles plastifiantes ou encore les produits de synthèse à base de mélanges liquides d'hydrocarbures saturés comme par exemple les produits commercialisés par la société TOTAL sous la dénomination GEMSEAL^{®} et en particulier le produit GEMSEAL^{®} 60 qui est un mélange isoparaffinique issu d'une coupe pétrolière totalement hydrogénée.

Dans le cadre de la présente invention, on préférera utiliser des huiles plastifiantes, de préférence des huiles minérales plastifiantes et en particulier des huiles minérales formées de composés de nature paraffinique ou naphténique ou de leurs mélanges dans des proportions variables.

Des huiles minérales plastifiantes particulièrement préférées sont formées de mélanges de composés de nature paraffinique et naphténique, et en particulier de tels mélanges dans lesquels la proportion de composés de nature paraffinique est majoritaire.

A titre d'exemple d'huile minérale plastifiante commerciale, on peut citer les produits commercialisés par la société SHELL sous la dénomination ONDINA^{®}.

Parmi ces produits, d'excellents résultats ont été obtenus avec les huiles commercialisées sous les dénominations ONDINA^{®} 917 ou ONDINA^{®} 919.

Comme indiqué précédemment, la quantité de plastifiant présente au sein de la matrice hydrophobe doit être spécifiquement choisie en fonction de la quantité d'élastomère.

Ainsi, pour 100 parties en poids d'élastomère, la matrice hydrophobe contiendra de 400 à 1 220 parties en poids, et de préférence de 600 à 900 parties en poids, de plastifiant, de préférence d'une huile plastifiante.

Pour augmenter le caractère et le toucher gras du mélange constitué par l'élastomère et le plastifiant, il peut être avantageux, dans le cadre de la présente invention, d'ajouter à ce mélange une quantité prédéterminée de vaseline.

Il s'agira de préférence d'une vaseline conforme à la Pharmacopée Française disponible commercialement.

La quantité de vaseline au sein de la matrice hydrophobe peut être généralement comprise entre 0 et 720 parties en poids, et de préférence de 150 à 450 parties en poids, pour 100 parties en poids d'élastomère.

Cependant, pour obtenir un pansement autoporté, il a été constaté que les deux conditions suivantes doivent être nécessairement respectées :
- la quantité totale de plastifiant et de vaseline doit être supérieure ou égale à 750 parties en poids, pour 100 parties en poids d'élastomère ;
- la quantité de vaseline doit être comprise entre 400 et 720 parties en poids lorsque la quantité de plastifiant est comprise entre 1000 et 1220 parties en poids.

La matrice hydrophobe qui vient d'être décrite constitue l'élément essentiel des compositions permettant de réaliser un pansement autoporté conforme à l'invention.

De telles compositions peuvent néanmoins comprendre des composés additionnels et en particulier des composés choisis parmi les antioxydants, les hydrocolloïdes et les agents actifs ou adjuvants couramment utilisés dans le domaine du traitement des plaies.

Ainsi, selon un mode de réalisation particulier, ces compositions comprennent un ou plusieurs agents antioxydants.

Les composés antioxydants ou stabilisants sont couramment utilisés pour assurer la stabilité des composés entrant dans la formulation des compositions pour pansement, en particulier vis-à-vis de l'oxygène, la chaleur, l'ozone ou les rayonnements ultraviolets.

Comme exemple d'agents antioxydants susceptibles d'être utilisés dans le cadre de la présente invention, on citera notamment les antioxydants phénoliques, comme en particulier les produits commercialisés par la société CIBA Specialty Chemicals sous les dénominations IRGANOX^{®} et en particulier les produits de référence IRGANOX^{®} 1010, IRGANOX^{®} 565 et IRGANOX^{®} 1076.

D'une façon générale, ces composés pourront être utilisés en une quantité de l'ordre de 0,05 à 1 % en poids, de préférence de 0,05 à 0,2 % en poids, rapportée au poids total de la composition.

D'une façon particulièrement avantageuse on utilisera le produit IRGANOX^{®} 1010 en une quantité comprise entre 0,05 et 0,1 % en poids, rapportée au poids total de la composition.

Selon un autre mode de réalisation de l'invention particulièrement préféré dans le cadre de la cicatrisation des plaies, les compositions formant le pansement selon l'invention comprennent des particules hydrophiles d'un hydrocolloïde.

Les particules d'hydrocolloïde sont de préférence dispersées de façon homogène au sein de la composition.

Par hydrocolloïde ou particules d'hydrocolloïde on entend désigner ici tout composé habituellement utilisé par l'homme du métier pour son aptitude à absorber les liquides aqueux tels que l'eau, le sérum physiologique ou les exsudats d'une plaie.

Dans le cadre de l'invention, ces composés seront utilisés pour favoriser un retrait indolore du pansement interface et maintenir un environnement humide au niveau de la plaie afin de favoriser la cicatrisation.

Comme hydrocolloïdes appropriés, on peut citer par exemple la pectine, les alginates, les gommes végétales naturelles comme en particulier la gomme de Karaya, les dérivés de cellulose tels que les carboxyméthylcelluloses et leurs sels de métal alcalin tels que le sodium ou le calcium, ainsi que les polymères synthétiques à base de sels de l'acide acrylique, connus sous l'appellation de « superabsorbants », comme par exemple les produits commercialisés par la société BASF sous la dénomination LUQUASORB^{®} 1003 ou par la société CIBA Specialty Chemicals sous la dénomination SALCARE^{®} SC91 ainsi que les mélanges de ces composés.

Les hydrocolloïdes préférés dans le cadre de la présente invention sont les sels de métal alcalin de la carboxyméthylcellulose, et en particulier la carboxyméthylcellulose de sodium (CMC).

La taille des particules d'hydrocolloïdes est généralement comprise entre 50 et 100 microns, notamment de l'ordre de 80 microns.

Les superabsorbants qualifiés de « microcolloïdes » car ils présentent une taille de particules inférieure à 10 micromètres peuvent également être utilisés.

La quantité de particules d'hydrocolloïde dispersées dans la composition sera généralement inférieure ou égale à 25 % et avantageusement de l'ordre de 2 à 20 % en poids, de préférence de 5 à 18 % en poids, de préférence encore de 10 à 15 % en poids rapportée au poids total de la matrice hydrophobe.

Le choix d'une quantité de particules d'hydrocolloïde comprise dans ces gammes de valeurs est importante pour la réalisation d'un pansement interface autoporté notamment afin d'éviter que la gélification de la composition lors de l'absorption des exsudats n'entraine la fermeture des trous traversants.

D'une façon particulièrement préférée, on utilisera une quantité d'hydrocolloïde suffisamment faible pour que le pansement interface obtenu présente une capacité d'absorption limitée, équivalente à celle du produit URGOTUL^{®}.

Selon encore un autre mode de réalisation, les compositions permettant de réaliser un pansement autoporté selon l'invention comprennent un ou plusieurs adjuvants et/ou agents actifs couramment utilisés dans le domaine du traitement des plaies et plus généralement dans le domaine pharmacologique.

De tels agents actifs sont en particulier des substances ayant un rôle favorable dans le traitement des plaies et notamment capables d'induire ou d'accélérer la cicatrisation durant la phase de détersion et/ou de granulation de la plaie.

La présence d'hydrocolloïdes au sein de la composition favorisera le relargage de ces agents actifs.

Parmi les autres agents actifs susceptibles d'être utilisés dans le cadre de l'invention, on citera par exemple les agents bactéricides ou bactériostatiques, les agents antidouleurs ou les anesthésiques locaux ainsi que les agents anti-inflammatoires.

D'une façon générale, les compositions formant les pansements conformes à l'invention peuvent comprendre de tels agents actifs en une quantité de 0,01 à 20 % en poids, de préférence de 1 à 15 % en poids et de préférence encore de 2 à 10 % en poids, rapportée au poids total de la composition.

Bien évidemment les trois modes de réalisation particuliers qui viennent d'être décrits peuvent être mis en oeuvre séparément ou selon l'une quelconque de leurs combinaisons. Les compositions qui viennent d'être décrites permettent de réaliser des pansements autoportés.

A cet effet, ces compositions seront formées en couche mince avec des trous traversants, disposés de préférence de façon répartie dans ladite couche.

Les trous traversants peuvent être réalisés par perforation ou poinçonnage d'une composition préalablement formée en couche mince, seule ou associée à un support provisoire ou à un film protecteur habituellement utilisé pour la fabrication de pansement, ou bien encore par une enduction tramée sur un support provisoire.

Pour plus de détails, on pourra se référer à la demande de brevet FR 2 936 158.

Alternativement, les pansements conformes à l'invention peuvent être fabriqués par coulage à chaud d'une composition telle que décrite précédemment sur une plaque gravée avec le motif retenu pour former des trous traversants, suivi d'un refroidissement et d'un démoulage.

D'une façon générale, les pansements conformes à l'invention présenteront une épaisseur comprise entre 0,4 mm et 2 mm, de préférence entre 0,5 mm et 1 mm, de préférence encore de l'ordre de 0,6 mm.

Les trous traversants peuvent être de géométrie quelconque et présenteront par exemple une section transversale circulaire, rectangulaire, trapézoïdale ou carrée.

Leur surface sera généralement comprise entre 0,25 et 5 mm².

Ces trous présenteront notamment un diamètre moyen compris entre 0,5 et 2 mm, de préférence de l'ordre de 1 mm, lorsque leur section transversale est circulaire.

Ces trous seront répartis, de préférence de façon régulière, avec une densité telle que la surface totale des trous représente entre 20 et 70 %, et de préférence entre 30 et 50 % de la surface totale du pansement.

Selon un mode de réalisation préféré, le pansement interface autoporté selon l'invention se présente sous la forme d'un filet aéré (ou grille) de préférence de maille carrée présentant :
- une épaisseur du filet comprise entre 0,5 et 2 mm ;
- une « largeur de fil » (largeur de l'espace entre deux trous consécutifs) comprise entre 1 et 10 mm, et de préférence entre 1 et 5 mm ;
- un grammage compris entre 200 et 1700 g/m², et de préférence compris entre 300 et 800 g/m².

Selon un mode de réalisation particulièrement préféré de l'invention, un tel pansement se présentera sous la forme d'un filet aéré à maille carrée présentant :
- une épaisseur du filet de 600 microns environ ;
- une largeur de fil de l'ordre de 2 mm ;
- un grammage de l'ordre de 450 g/m².

De façon classique, un tel pansement peut être associé à un support protecteur déposé sur chacune de ses faces opposées afin de protéger le pansement de l'environnement extérieur, lesdits protecteurs étant destinés à être retirés immédiatement avant usage.

De tels supports protecteurs sont bien connus de l'homme du métier et peuvent être par exemple constitués d'un film de polyester siliconé.

Alternativement, et afin de faciliter encore la manipulabilité du pansement interface autoporté selon l'invention, celui-ci pourra être associé à un système protecteur tel que décrit dans la demande WO 2008/145 884 formé d'une feuille unique recouvrant les deux faces opposées du pansement et facilitant son application sur la plaie.

L'invention sera illustrée par les exemples non limitatifs suivants.

### EXEMPLES 1 A 3

### Préparation d'un pansement interface autoporté selon l'invention

Dans un malaxeur à bras en Z, on introduit successivement sous agitation à une température de consigne de 90°C le plastifiant et l'hydrocolloïde et on malaxe jusqu'à l'obtention d'un mélange homogène.

Après avoir porté la température de consigne à 140°C, on introduit sous agitation l'élastomère et un anti-oxydant, puis on malaxe jusqu'à l'obtention d'un mélange homogène.

La vaseline est alors introduite sous agitation à 140°C en deux fois jusqu'à l'obtention d'un mélange homogène.

Le mélange ainsi obtenu est coulé à chaud à une température de l'ordre de 120-130°C sur une plaque plane gravée formant par exemple l'empreinte d'un filet ou grille à maille carrée.

Après refroidissement et démoulage, on obtient le pansement attendu sous forme d'un filet à maille carrée présentant une épaisseur de 600 µm environ, une taille de maille de l'ordre de 2 mm, une épaisseur de l'ordre de 0,6 mm et un grammage de l'ordre de 450 g/m².

Les pansements ainsi réalisés ont été placés entre deux films protecteurs provisoires en polyester siliconé de 50 µm d'épaisseur.

Les pansements des exemples 1 à 3 ont été élaborés à l'aide des constituants suivants, dans les proportions en poids mentionnées dans le tableau 1 :
- élastomère: copolymère séquencé de poly(styrène-éthylène-butylène-styrène) (en abrégé SEBS):
   - KRATON^{®} G1654 (exemple 1) ;
   - KRATON^{®} G1651 (exemples 2 et 3)
   - plastifiant: huile minérale Ondina^{®} 917 commercialisée par la société SHELL
   - anti-oxydant: IRGANOX^{®} 1010 commercialisé par la société CIBA Specialty Chemicals
   - vaseline: vaseline Codex A commercialisée par la société AIGLON - hydrocolloïde: Carboxyméthylcellulose sodique : CMC BLANOSE^{®} 7H4XF commercialisée par la société ASHLAND.

**TABLEAU I**

| COMPOSITION | | EXEMPLE 1 | | EXEMPLE 2 | | EXEMPLE 3 | |
|---|---|---|---|---|---|---|---|
| | | % | Parties | % | Parties | % | Parties |
| ELASTOMERE | KRATON G 1651 | | | 4,9 | 100,00 | 4,9 | 100,00 |
| | KRATON G 1654 | 8,3 | 100,00 | | | | |
| PLASTIFIANT | | 61,5 | 740,96 | 55 | 1 122,45 | 45 | 918,37 |
| VASELINE | | 15 | 180,721 | 25 | 510,20 | 35 | 714,29 |
| ANTIOXYDANT | | 0,2 | 2,41 | 0,1 | 2,04 | 0,1 | 2,04 |
| HYDROCOLLOIDE | | 15 | 180,72 | 15 | 306,12 | 15 | 306,12 |
| | | 100 | 1 204,82 | 100 | 2 040,82 | 100 | 2 040,82 |

## Revendications

1. Pansement interface autoporté formé d'une couche mince d'une composition comprenant une matrice hydrophobe et comportant des trous traversants, **caractérisé en ce que** ladite matrice hydrophobe comprend :
- pour 100 parties en poids d'un copolymère tribloc styrène-oléfine saturée-styrène présentant une viscosité comprise entre 0,2 et 2 Pa.s, telle que mesurée dans une solution à 10 % (masse/masse) dans le toluène ;
- de 400 à 1 220 parties en poids d'un plastifiant, de préférence une huile plastifiante ; et
- de 0 à 720 parties en poids de vaseline ;
étant en outre précisé que :
- la quantité totale de plastifiant et de vaseline est supérieure ou égale à 750 parties en poids ;
- la quantité de vaseline est comprise entre 400 et 720 parties en poids lorsque la quantité de plastifiant est comprise entre 1 000 et 1 220 parties en poids.

2. Pansement selon la revendication 1, **caractérisé en ce que** la matrice hydrophobe précitée comprend, pour 100 parties en poids du copolymère précité :
- de 600 à 900 parties en poids d'un plastifiant, de préférence une huile plastifiante ; et
- de 150 à 450 parties en poids de vaseline.

3. Pansement selon la revendication 1 ou 2, **caractérisé en ce que** la composition précitée comprend, en outre, des particules d'hydrocolloïde en une quantité inférieure ou égale à 25 % en poids rapportée au poids total de ladite matrice hydrophobe.

4. Pansement selon l'une des revendications 1 à 3, **caractérisé en ce que** la composition précitée comprend, en outre, une ou plusieurs substances choisies parmi les substances ayant un rôle favorable dans le traitement des plaies, les agents bactéricides ou bactériostatiques, les agents anti-douleurs ou les anesthésiques locaux ainsi que les agents anti-inflammatoires, en une quantité comprise entre 0,01 et 20 % en poids, de préférence entre 1 et 15 % en poids, rapportée au poids total de la composition.

5. Pansement selon l'une des revendications 1 à 4, **caractérisé en ce que** la composition précitée comprend, en outre, un ou plusieurs agents anti-oxydant en une quantité de 0,05 à 1 % en poids, de préférence de 0,05 à 0,2 % en poids, rapportée au poids total de la composition.

6. Pansement selon l'une des revendications 1 à 5, **caractérisé en ce qu'**il se présente sous la forme d'un filet aéré ou grille présentant une épaisseur comprise entre 0,4 et 2 mm, de préférence entre 0,5 mm et 1 mm.

7. Pansement selon l'une des revendications 1 à 6, **caractérisé en ce qu'**il comprend des trous traversants, chaque trou pouvant être de géométrie quelconque et dont la surface est comprise entre 0,25 et 5 mm² ; la surface totale desdits trous représentant entre 20 et 70 %, de préférence entre 30 et 50 % de la surface totale du pansement.

## Patentansprüche

1. Selbsttragender Grenzschicht-Verband, der von einer dünnen Schicht aus einer Zusammensetzung gebildet ist, die eine hydrophobe Matrix umfasst und Durchgangslöcher aufweist, **dadurch gekennzeichnet, dass** die hydrophobe Matrix umfasst:
- für 100 Gewichtsteile eines Dreiblock-Copolymers Styrol-gesättigtes Olefin-Styrol mit einer Viskosität zwischen 0,2 und 2 Pa.s, gemessen in einer 10 %igen (Masse/Masse) Lösung in Toluol,
- 400 bis 1220 Gewichtsteile eines Weichmachers, vorzugsweise eines weichmachenden Öls, und
- 0 bis 720 Gewichtsteile Vaseline,
wobei weiterhin präzisiert ist, dass:
- die Gesamtmenge an Weichmacher und Vaseline mehr als oder gleich 750 Gewichtsteile beträgt,
- die Menge an Vaseline zwischen 400 und 720 Gewichtsteile beträgt, wenn die Menge an Weichmacher zwischen 1000 und 1220 Gewichtsteile beträgt.

2. Verband nach Anspruch 1, **dadurch gekennzeichnet, dass** die vorgenannte hydrophobe Matrix für 100 Gewichtsteile des vorgenannten Copolymers:
- 600 bis 900 Gewichtsteile eines Weichmachers, vorzugsweise eines weichmachenden Öls, und
- 150 bis 450 Gewichtsteile Vaseline umfasst.

3. Verband nach Anspruch 1 oder 2, **dadurch gekennzeichnet, dass** die vorgenannte Zusammensetzung ferner Hydrokolloid-Partikel in einer Menge von weniger als oder gleich 25 Gew.-%, bezogen auf das Gesamtgewicht der hydrophoben Matrix, umfasst.

4. Verband nach einem der Ansprüche 1 bis 3, **dadurch gekennzeichnet, dass** die vorgenannte Zusammensetzung ferner eine oder mehrere Substanzen, die aus den Substanzen mit einer bei der Wundbehandlung günstigen Funktion, den Bakteriziden oder Bakteriostatika, den schmerzstillenden Mitteln oder den Lokalanästhetika sowie den entzündungshemmenden Mitteln ausgewählt sind, in einer Menge zwischen 0,01 und 20 Gew.-%, vorzugsweise zwischen 1 und 15 Gew.-%, bezogen auf das Gesamtgewicht der Zusammensetzung, umfasst.

5. Verband nach einem der Ansprüche 1 bis 4, **dadurch gekennzeichnet, dass** die vorgenannte Zusammensetzung ferner ein oder mehrere Antioxidantien in einer Menge von 0,05 bis 1 Gew.-%, vorzugsweise von 0,05 bis 0,2 Gew.-%, bezogen auf das Gesamtgewicht der Zusammensetzung, umfasst.

6. Verband nach einem der Ansprüche 1 bis 5, **dadurch gekennzeichnet, dass** er in Form eines belüfteten Netzes oder Gitters, das eine Dicke zwischen 0,4 und 2 mm, vorzugsweise zwischen 0,5 mm und 1 mm aufweist, vorliegt.

7. Verband nach einem der Ansprüche 1 bis 6, **dadurch gekennzeichnet, dass** er Durchgangslöcher umfasst, wobei ein jedes Loch eine beliebige Geometrie aufweisen kann und seine Fläche zwischen 0,25 und 5 mm² beträgt, wobei die Gesamtfläche der Löcher zwischen 20 und 70 %, vorzugsweise zwischen 30 und 50 % der Gesamtfläche des Verbands ausmacht.

## Claims

1. A self-supporting interface dressing formed from a thin layer of a composition comprising a hydrophobic matrix and having through holes, **characterized in that** said hydrophobic matrix comprises:
- per 100 parts by weight of a styrene-saturated olefin-styrene triblock copolymer having a viscosity between 0.2 and 2 Pa.s, as measured in a 10% (weight/weight) solution in toluene;
- from 400 to 1220 parts by weight of a plasticizer, preferably a plasticizing oil; and
- from 0 to 720 parts by weight of petroleum jelly;
it being specified moreover that:
- the total amount of plasticizer and petroleum jelly is greater than or equal to 750 parts by weight;
- the amount of petroleum jelly is between 400 and 720 parts by weight when the amount of plasticizer is between 1000 and 1220 parts by weight.

2. The dressing as claimed in claim 1, **characterized in that** the abovementioned hydrophobic matrix comprises, per 100 parts by weight of the abovementioned copolymer:
- from 600 to 900 parts by weight of a plasticizer, preferably a plasticizing oil; and
- from 150 to 450 parts by weight of petroleum jelly.

3. The dressing as claimed in claim 1 or 2, **characterized in that** the abovementioned composition comprises, in addition, hydrocolloid particles in an amount of less than or equal to 25% by weight relative to the total weight of said hydrophobic matrix.

4. The dressing as claimed in one of claims 1 to 3, **characterized in that** the abovementioned composition comprises, in addition, one or more substances selected from substances that have a favorable role in the treatment of wounds, bactericidal or bacteriostatic agents, painkillers or local anesthetics, and also antiinflammatories, in an amount of between 0.01 and 20% by weight, preferably between 1 and 15% by weight, relative to the total weight of the composition.

5. The dressing as claimed in one of claims 1 to 4, **characterized in that** the abovementioned composition comprises, in addition, one or more antioxidants in an amount of from 0.05% to 1% by weight, preferably from 0.05% to 0.2% by weight, relative to the total weight of the composition.

6. The dressing as claimed in one of claims 1 to 5, **characterized in that** it is in the form of a breathable net or grid having a thickness of between 0.4 and 2 mm, preferably between 0.5 mm and 1 mm.

7. The dressing as claimed in one of claims 1 to 6, **characterized in that** it comprises through holes, it being possible for each hole to be of any geometry and the surface area of which is between 0.25 and 5 mm²; the total surface area of said holes representing between 20% and 70%, preferably between 30% and 50% of the total surface area of the dressing.
